# EUROPEAN PATENT APPLICATION

(11) **EP 0 553 534 A1**
(43) Date of publication of application: **04.08.1993**
(21) Application number: 92307269.8
(22) Date of filing: 07.08.1992
(51) Int. Cl.: A61J 1/00, A61M 35/00

(54) **A tubular container and packages for the same**

(30) Priority: 28.01.1992 US 827068
(71) Applicant: Tsao, Chien-Hua, Taipei (TW)
(72) Inventor: Tsao, Chien-Hua, Taipei (TW)
(74) Representative: Arthur, Bryan Edward

(57) **Abstract**

A tubular container (10) and its packages (150), including a tubule (11) filled with a desired liquid (14) in an appropriate volume and having a first open end (12) wrapped with or connected to a soft absorbent (26), a second open end (15) sealed by a detachable sealing element (13) in a manner that the surface tension of the liquid (14) in the tubule (11) must be lower than the atmospheric pressure so that the liquid (14) will not flow out before the sealing element (13) is detached.

## Description

The present invention relates to a tubular container and packages for the same, particularly to a tubular container with a closed end in which liquid may be stored to facilitate carrying.

In our daily life, merbromin solution, povidon solution, methylrosaniline chloride solution, alcohol, cream, syrup, astringent, colonge, facial cream, seasoner, and other solutions are used frequently, each of which is packed in a considerable large volume, and it is inconvenient to carry it, and to dispose of its container as well, for instance:
a. The user has to pay a lot for buying of such a considerable large volume, which indeed part of which has to be discarded later because of expiry or deterioration, and hence it is a waste as well as an economic burden to the consumer;
b. It is very inconvenient to bring it in considerable large bottle, particularly while shopping or traveling, it is impossible to bring all required at a time;
c. Use of it requires cotton bud, spoon or brush, which means an inconvenience; and
d. Deterioration happens due to prolonged exposure to the air after opening of the bottle, particularly in the case of medicine, cotton bud will bring bacteria into the medicine by absorption it with the cotton bud, and hence the medical effect of the medicine is affected.

Therefore, there has been a need of compact and convenient tubular container and its packages.

The main object of the present invention is to provide a tubular container to store liquid so that it can be placed and identified separately in a package to become a easy-to-bring package.

Another object of the present invention is to provide a tubular container including a tubule with an open end wrapped with soft absorbent, and another open end sealed with a sealing element so that by detaching the sealing element, the atmospheric pressure can force the liquid to flow out of the tubule, and hence provide a simple, low-cost and easy-to-make container.

Another object of the present invention is to provide a tubular container and its packages with tubules to hold different liquid for different application.

The tubular container according to the present invention comprises mainly a hollow tubule having an open end sealed with a sealing element and another open end for filling in and flowing out of liquid. The latter is wrapped with or connect to a soft absorbent, spoon or brush, and the surface tension of the liquid in the tubule is lower than the atmospheric pressure so that the liquid can be retained in the tubule in spite of the location of the latter open end. By detaching the sealing element, liquid in the tubule flows out to the soft absorbent, spoon or brush. Hence, the liquid can be applied to any desired place easily and conveniently.

Many advantages of the present invention may be ascertained from a reading of the specification and the claims in conjunction with the drawings, in which:
Fig. 1 is a sectional view of the first embodiment of a tubular container according to the present invention;
Fig. 2 is a sectional view of the second embodiment of a tubular container according to the present invention;
Fig. 3 is a magnified view of the right side of the second embodiment shown at Fig. 2 when no external force is applied;
Fig. 4 is a magnified view of the right side of the second embodiment shown at Fig. 2 when a external force is applied;
Fig. 5 is a sectional view of the third embodiment of a tubular container according to the present invention;
Fig. 6 is a partial sectional view of the fourth embodiment of a tubular container according to the present invention;
Fig. 7 is a partial sectional view of the fifth embodiment of a tubular container according to the present invention;
Fig. 8 is a partial sectional view of the sixth embodiment of a tubular container according to the present invention;
Fig. 9 is a sectional view of the seventh embodiment of a tubular container according to the present invention;
Fig. 10 is a partial magnified perspective and fragmented view of the eighth embodiment of a tubular container according to the present invention;
Fig. 11 is a top view of the tubular container shown in Fig. 10;
Fig. 12 is a longitudinal sectional view of the sealing element in Fig. 10 after turning for a certain degree;
Fig. 13 is a sectional view of the ninth embodiment of a tubular container according to the present invention;
Fig. 14 is a sectional view of the tenth embodiment of a tubular container and its packages according to the present invention;
Fig. 15 is a sectional view of the eleventh embodiment of a tubular container and its packages according to the present invention;
Fig. 16 is a magnified perspective view of a part of the embodiment shown in Fig. 15;
Fig. 17 is the twelve embodiment of a tubular container and its packages similar to that shown in Fig. 15;
Fig. 18 is a magnified perspective view of a part of the embodiment shown in Fig. 17; and
Fig. 19 is a sectional view of another embodiment of some soft absorbent and their package according to the present invention.

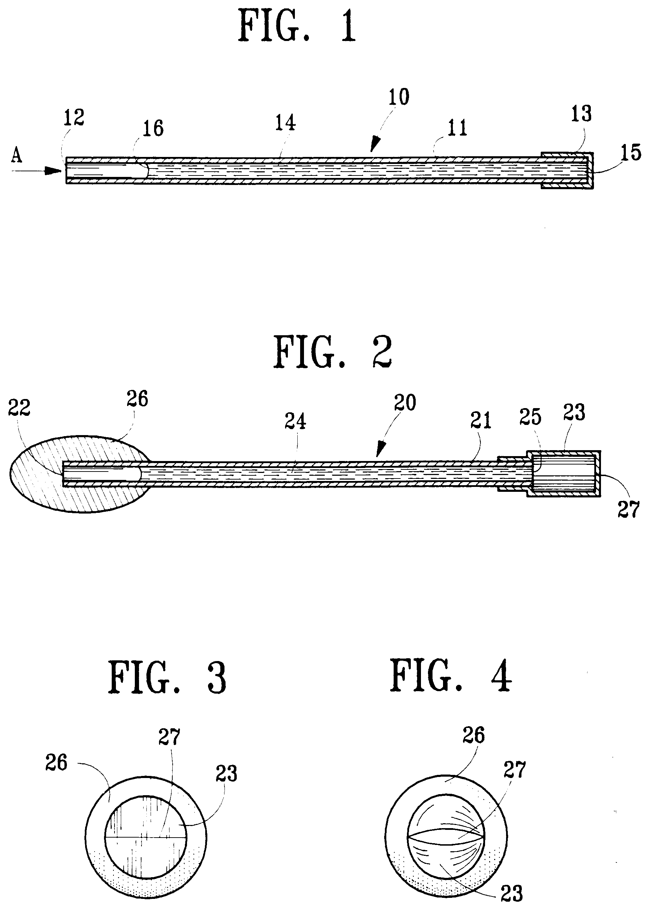

As shown in Fig. 1, a cross sectional view of the first embodiment of a tubular container according to the present invention, the tubular container (10) comprises a hollow tubule (11) having a front open end (12) and a rear open end (15) sealed with a sealing element (13) to store liquid within the hollow tubule (11). The sealing element is generally a round sleeve made of nontoxic rubber so that the good elasticity of rubber can airtightly seal the rear open end (15) to isolate the liquid from the atmosphere at that open end (15). The pressure at the surface (16) of the liquid (14) must be lower than the atmospheric pressure A which acts on the surface (16) of the liquid (14) in the tubule (11) through the front open end (12) so that the liquid will not flow out of the tubule (11) before the sealing element (13) is removed from the rear open end (15). To apply the liquid (14) from the tubule (11), only the sealing element has to be removed to make both ends (12 and 15) of the tubule (11) open and made atmospheric pressure to enter the tubule (11) from both ends (12 and 15). The pressure in the tubule (11) is thus balanced, and the liquid (14) kept in the tubule (11) can flow out by its own gravity after removal of the sealing element (13).

Please refer to Figs. 2 thru 4 which illustrate the second embodiment of the tubular container according to the present invention. The tubular container (20) comprises a hollow tubule (21) to store medicine or cosmetic in liquid form, having an front open end (22) wrapped with a soft and permeable absorbent (26), and a rear open end (25) sealed with a sealing element (23) in the form of a sleeve. The sealing element (23) has a prolonged slit (27) which is maintained air and water tight if no external force is applied to it, as shown in Fig. 3, so that the liquid (24) in the tubule (21) will not flow out upon effect of the atmospheric pressure. To use the liquid (24), the sealing element (23) is slightly squeezed at its outer side to open the slit (27) by squeezing as shown in Fig. 4 so that external air can enter the tubule (21) freely, a slight slanting of the tubule (21) can cause the liquid (24) to flow out of the tubule (21) by its own gravity from the open end (22), and consequently the liquid (24) is absorbed by the soft absorbent (22) to ease application. The sealing element (23) is for closing of the end (22) and control of flow rate as well.

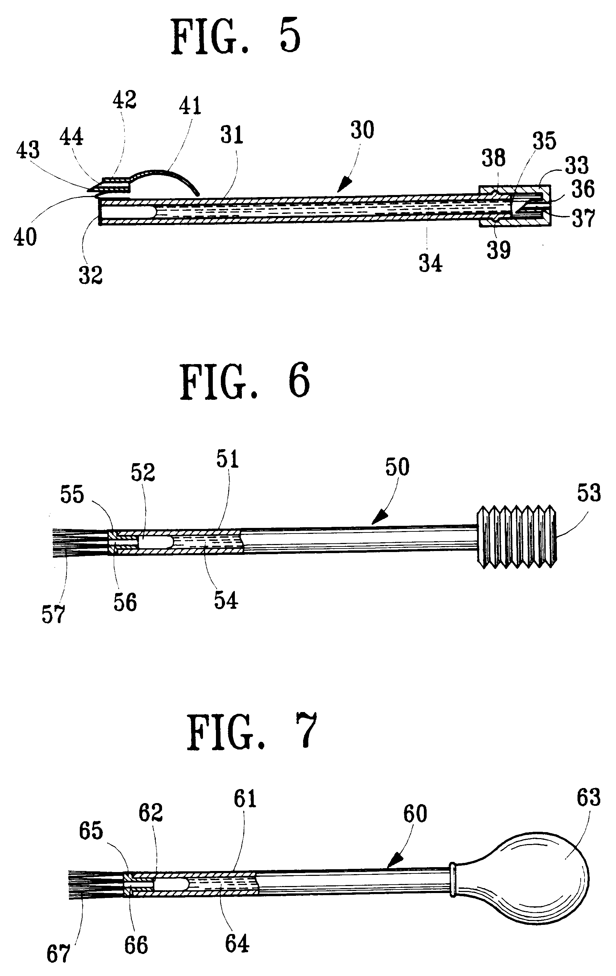

As shown in Fig. 5, which shows the third embodiment of the tubular container according to the present invention, the tubular container (30) comprises a hollow tubule (31) with two open ends (32 and 35) each sealed with a film or tin foil to maintain airtight condition. The end (35) is incorporated with a cylindrical like piercing element (33) having a hollow tip (37) formed at the center of the front side of its base (36), and an annular color (38) on its internal circumference at an appropriate location for fitting to an annular groove (39) formed around the outer circumference at the end of tubule (31). The other end (32) of the tubule (31), at sealing condition, has a connecting element (40) connecting to a freely rotatable spoon (41) fixed to a fixing block (42) which is designed with a size just for inserting into the tubule (32). The fixing block is formed with a piecing element (43) and a central air passage (44). With such a design, the liquid (particularly seasoner) is retained within the tubule (31) before the film or tin foil at the end (32 or 35) is broken, the piercing tip (33) will not puncture the film or tin foil when its annular collar (38) is fitted to the annular groove (39). To use the liquid, a slight force is applied to the piercing element (33) to disengage the annular collar (38) from the annular groove (39) so that the hollow tip (37) can puncture the film or tin foil at the end (35) to let external air to enter the tubule (31), and then, turning the spoon (41) outward and insert its fixing block (42) into the tubule (31), its piercing tip (33) puncture the film or tin foil sealing the end 32) and let the external air to enter the tubule (31) via the air passage (44) to keep a balanced pressure in the tubule (32). At such a condition, a slight slanting of the tubule (31) can cause the liquid to flow to the spoon (41) which is serving to receive the liquid and to stir as well.

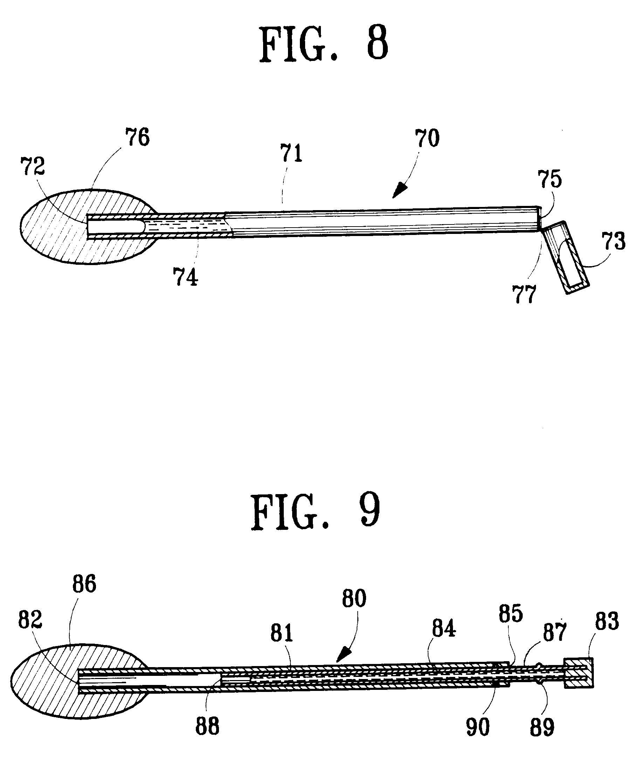

Please refer to Figs. 6 and 7 which illustrate the fourth and the fifth embodiments of the tubular container according to the present invention respectively, the tubular container (50 or 60) comprises a tubule (51 or 61) to contain a liquid (54 or 64), particularly cosmetic in liquid form, having an open end (51 or 61) to which a brush seat (55 or 65) with a central air passage (56) is inserted, and another open end (52 or 62) sealed with a bellows-like sealing element (53) or a ball-like sealing element (54) so that by squeezing the bellows-like sealing element (65), the liquid (54 or 64) can be squeezed and forced to flow out of the tubule (51 or 61) through the open end (52 or 62), and consequently the liquid (54 or 64) is absorbed by a brush (57 or 67) on the brush seat (55 or 65) to ease application to skin of its user.

Please refer to Fig. 8, the sixth embodiment of a tubular container according to the present invention, the tubular container (70) comprises a hollow tubule (71) having a front open end (72) end wrapped with a soft absorbent (76) and a rear open end (75) with a snap portion sealed by a sealing element (73). The snap portion can be snapped easily to allow air to enter the tubule (71) so that the liquid can flow toward the soft absorbent (76) at the open end (72), and absorbed by the soft absorbent (76) to ease application.

As shown in Fig. 9, the seventh embodiment of a tubular container (80) according to the present invention comprises a hollow tubule (81) having a front open end (82) wrapped with a soft absorbent (86) and a rear open end (88), and an inner tube (87) having an end sealed with a sealing element (83) and an open end (88) for preservation of liquid. An annular collar (89) is formed near the sealing element (83) for fitting the inner tube (87) to the tubule (71) by engaging it with an annular groove (90) formed around the circumference of the tubule (81). By removing the sealing element (83), the liquid (84) in the inner tube (87) can flow through the open end (82) by its gravity, and then completely absorbed by the soft absorbent (86) to ease application. Therefore, it is a structure to permit separate and simultaneous production of the tubule (81) and the inner tube (75) filled with the desired liquid.

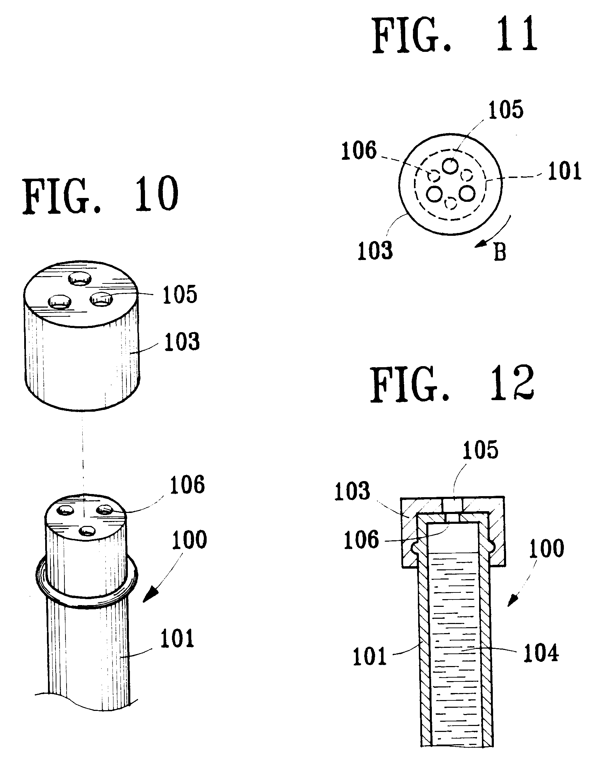

Please refer to Figs. 10 thru 12 which illustrate the eighth embodiment of a tubular container according to the present invention, the tubular container (100) consists of a hollow tubule (101) for storage of liquid (104). The tubule (101) has a front open end sealed with a cylindrical sealing element (103) formed with a plurality of vents (105) corresponding to a plurality of vents (106) at the end of the tubule (101), but such vents (105) and (106) are not aligned with each other normally as shown in Fig. 11. By turning the sealing element (103) for a certain degree to align the vents (105) with the vents (106) as shown in Fig. 12, the liquid (104) can flow out from an open end (not shown) of the tubule (101) by its gravity because of atmospheric pressure.

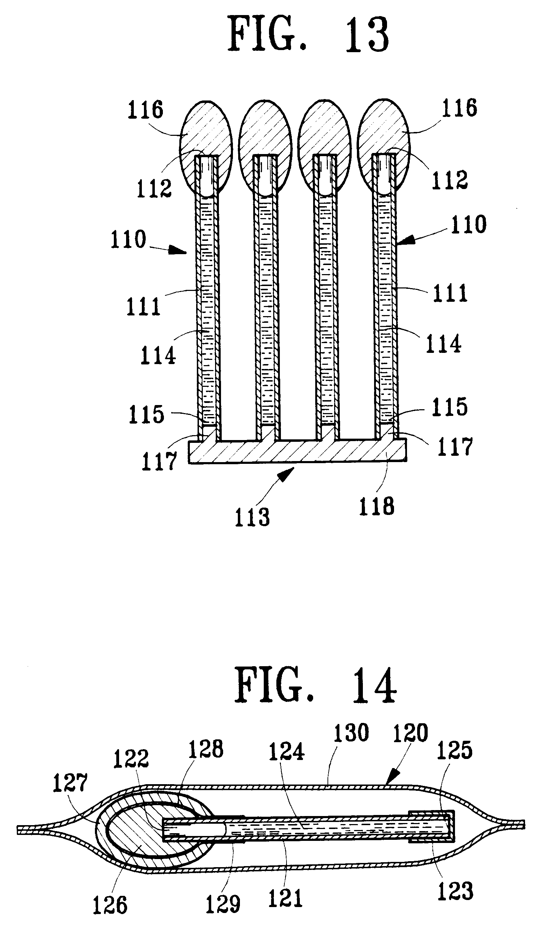

Please refer to Fig. 13, a plurality of tubular containers (11) according to the present invention can be fitted to a serial sealing element (113). The serial sealing component (113) has a plurality of independent projected sealing elements (117) and a connecting element to connect the sealing elements (117) together. Each of the tubular containers (110) has a hollow tubule (111) filled with a desired liquid (114) for a certain purpose. For instance, if the tubular containers (110) are for holding of seasoners, each the tubules (111) may be designated to store cream, syrup, sauce, and the like to ease carriage.

Please refer to Fig. 14 for another embodiment of the tubular container and their package according to the present invention, the tubular container with multiple absorbents (120) has a hollow tubule (121) with two open ends (122 and 125), one of which (125) is sealed with a removable sealing element and the other end (122) is wrapped with a permeable soft absorbent (126) which is covered by another layer of permeable soft absorbent (127) premoistened with a liquid different from that stored in the tubule (121). These two permeable soft absorbents (126 and 127) are separated by a separating element (128) such as permeable tape, which has a tip extended beyond the permeable soft absorbent (127) so that its removal can separate the permeable soft absorbent (127) by pulling out the extended tip (129). The permeable soft absorbents according to the present invention can be made in three or more layers each for a particular purposes. For instance, if the tubular container is for use in first-aid kits, the tubule (121) is filled with povidon solution (124), the outer absorbent is saturated with hydrogen peroxide or alcohol for sterilization. In its application, the tip (129) is pulled out to remove the outermost absorbent (127) and expose the inner absorbent (128), and then the sealing element (123) is removed so that the povidon solution (124) can flow to the absorbent (128) and then it can be applied to wound portion. In order to prevent from drying or deterioration of the liquid absorbed by the absorbent (127), the tubular container with multiple absorbents is preferably packed in a vacuum package (130) which can be opened easily.

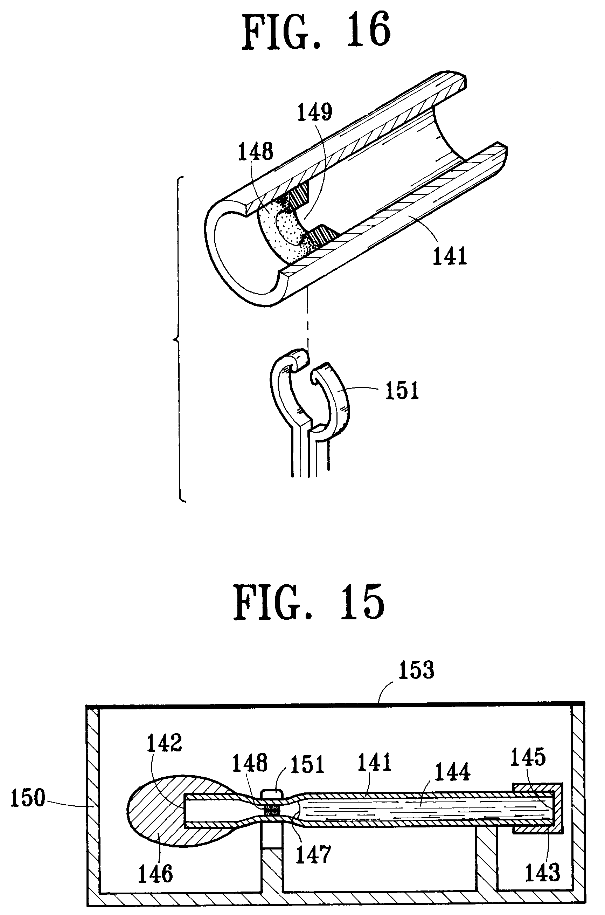

Please refer to Figs. 15 and 16, when the volume stored is large or the specific gravity is large so that the pressure exists at surface (147) of the liquid (144) in a tubule (141) is greater than the atmospheric pressure, the liquid (144) in the tubule (141) will flow out gradually from an open end (142). Therefore, for storage of liquid in considerably large volume or liquid of high specific gravity, both ends of the tubule (141) must be sealed. An annular elastic body (148) with a small opening (149) is placed on the inner wall of the open end (142) of the tubule (141) near a soft absorbent layer (146). Another open end (145) of the tubule (141) is sealed with a detachable sealing element (143). The tubule (141) is placed within a hard package (150) designed with a C-like clip to squeeze the tubule (141) at the annular elastic body (148) so that the opening (149) of the annular elastic body (148) is sealed, as shown in Fig. 15, and consequently to prevent from flowing out of liquid from the tubule (141). Upon removal the tubule (141) from the package, the annular elastic body (148) is released and the opening (149) resumes its normal opening condition as shown in Fig. 16 to permit flowing out of liquid from the tubule (141) and absorption of the liquid by the soft absorbent (146). The upper opening of the hard package (150) is sealed by a detachable aluminum foil or film (153). The hard package (150) may be designed with a size to keep a plurality of the tubular containers.

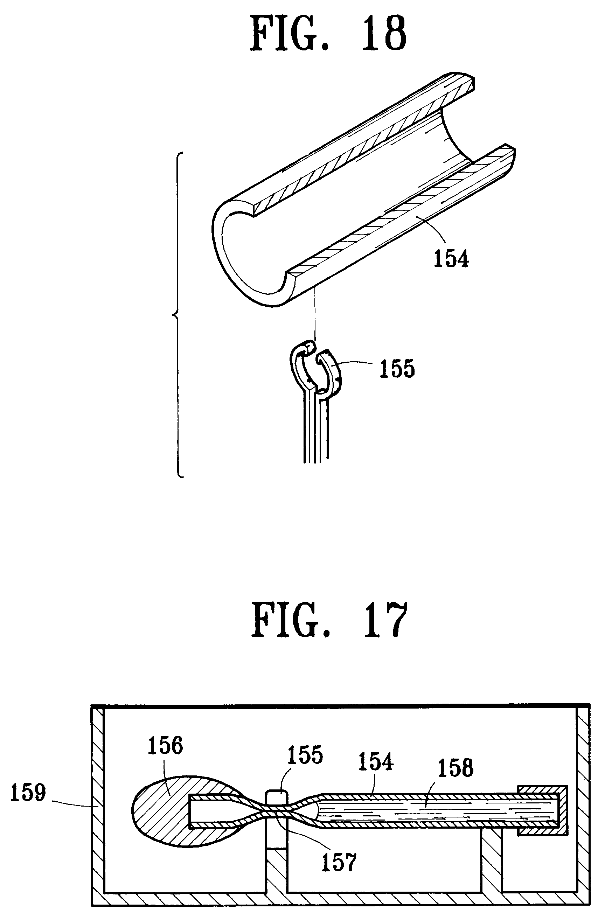

Please refer to Figs. 17 and 18 for the twelveth embodiment of the tubular container and their package according to the present invention, the tubular container is similar to the eleventh embodiment shown in Figs. 15 and 16 except the tubule (154) is made of compressible soft and elastic material so that a C-like clip (155) having a diameter smaller than its diameter can squeeze it as an end with soft absorbent (156) to form a sealed section (157) to prevent from flowing out of liquid from the tubule (154). Upon removal of the tubule (154) from the C-like clip (155), the sealed section (157) resumes its normal opening condition to become an open end (158) as shown in Fig. 18 for flowing out of the liquid. The C-like clip (155) is formed by extension from bottom of a hard package (159).

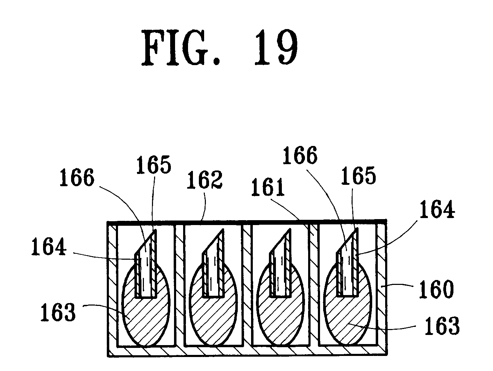

The soft absorbent to wrap the open end of the hollow tubule according to the present invention, as shown in Fig. 19, can be packed separately in a package (160). The soft absorbent (163) is adhered to a holder (164) with a piercing part (165) and a central vent (166). Each package (160) can hold a plurality of soft absorbents (163). A partitioning board (161) is used to separated two consecutive soft absorbents (163). The upper opening of the package (160) is sealed by a detachable aluminum foil or film (162). The soft absorbent (163) in the vacuum package hereof can be used in the first embodiment of a tubular container as shown in Fig, 1, or other tubular container with an end sealed with an aluminum foil or film and another end sealed by a sealing element for large volume storage.

The soft absorbent according to the present invention is preferably made of natural cotton. Other materials suitable for making of the soft absorbent are seed fiber, vegetable fiber, animal fiber, staple fiber, artificial raw material and other soft plastic material with good absorbency and acceptable elasticity. The material to make the soft absorbent must be nontoxic, and easy to sterilize and handle.

The liquid referred in the present invention include general medicine for treatment of wound in family, such as hydrogen peroxide solution, merbromin solution, acrinol solution, methylrosaniline chloride solution and povidon solution. It also can be other fluid such as perfume to serve as sample, alcohol for cleaning of magnetic head. The tubular container may be disposable to prevent from secondary infection.

As described above, the tubular container and packages for the same according to the present invention includes a hollow tubule for storage of liquid so that by removing its sealing element, the liquid can be absorbed by a soft absorbent wrapping its open end to ease application. The dimension and shape of the tubular container is substantially like a conventional cotton bud. Because its structure is practical, it is easy to make, its production cost is low. The tubular container is packed in an airtight package to isolate the container from the environment. Each packing can hole a plurality of tubular containers with the same or different contents. It is suitable for indoor or outdoor use, and it can be designed to meet different requirements.

Many changes and modifications in the above described embodiments of the invention can, of course, be carried out without departing from the scope hereof. Accordingly, to promote the progress in science and the useful arts, the invention is disclosed and is intended to be limited only by the scope of the appended claims.

## Claims

1. A tubular container comprising
- a hollow tubule with a first and a second open ends;
- a detachable sealing element to seal the second open end of the hollow tubule; and
- a liquid filled in the tubule having a surface tension lower than the atmospheric pressure so that the liquid is maintained in the hollow tubule and will not flow out through the first open end before the sealing element is detached.

2. A tubular container as claimed in Claim 1 wherein the sealing element is a flexible, airtight cylindrical sleeve made of nontoxic rubber.

3. A tubular container as claimed in Claim 1 wherein the sealing element has a prolonged slit which is maintained air and water tight if no external force is applied to it, but can be forced to open whenever the sealing element is squeezed so that external air can enter the hollow tubule through the second open end.

4. A tubular container as claimed in Claim 1 wherein the sealing element comprising a film to seal the second open end, a sliding piercing element connected to the hollow tubule and having a hollow tip to break the film by sliding of the piercing element to the hollow tubule axially in order to let external air to enter the hollow tubule through the hollow tip.

5. A tubular container as claimed in Claim 1 wherein the sealing element to seal the second open end is a bellows-like structure so that by squeezing the bellows-like sealing element, the liquid in the hollow tubule is subject to a pressure and is forced to flow out of the hollow tubule.

6. A tubular container as claimed in Claim 1 wherein the sealing element to seal the second open end is a ball-like structure so that by squeezing the ball-like sealing element, the liquid in the hollow tubule is subject to a pressure and is forced to flow out of the hollow tubule.

7. A tubular container as claimed in Claim 1 wherein the sealing element has detachable portion.

8. A tubular container as claimed in Claim 1 further comprising an outer tube just to hold the hollow tubule and having a first and a second open ends, in which the hollow tubule is inserted into the outer tube through the second open end of the outer tube, and the first open end of the outer tube is wrapped with a soft absorbent.

9. A tubular container as claimed in Claim 1 wherein the sealing element to seal the second open end includes an end face connected to the hollow tubule and having a plurality of interior vents, a cylindrical element fitted to the hollow tubule tightly and having a plurality of external vents and designed to align or discontinue alignment of these interior and external vents by turning of the cylindrical element.

10. A tubular container as claimed in Claim 1 further comprising a serial sealing component composed of a plurality of independent projected sealing elements and a connecting element to connect these sealing elements, each of which is to seal the second open end of a hollow tubule by fitting thereto tightly.

11. A tubular container as claimed in Claim 1 wherein the first open end of the hollow tubule is wrapped with a soft absorbent made of good absorbency, permeable and nontoxic material.

12. A tubular container as claimed in Claim 11 further comprising at least a layer of nontoxic permeable soft absorbent over the said soft absorbent to absorb a liquid different from that stored in the hollow tubule, and a detachable and permeable separating element between two consecutive soft absorbents.

13. A tubular container as claimed in Claim 12 wherein the tubular container is airtightly packed in a package.

14. A tubular container as claimed in Claim 1 wherein the hollow tubule is connected to a freely rotatable spoon by a connecting element, the spoon includes a fixing block with a piercing part and a central air passage, and the fixing block is fitted in the hollow tubule through the first open end.

15. A tubular container as claimed in Claim 1 wherein the first open end is incorporated with a brush seat having a central air passage and a brush fixed thereto.

16. A tubular container as claimed in Claim 1 wherein the tubule is made of compressible soft and elastic material and packed in a hard package having a C-like clip to retain and squeeze the tubule at an open end to form a sealed section.

17. A tubular container as claimed in Claim 16 wherein the tubuler has an annular elastic body with a small opening at the sealed section so that the small opening of the annular elastic body can be squeezed by the C-like clip to maintain closed condition.

18. A tubular container as claimed in Claim 1 further comprising a soft absorbent made of good permeable and nontoxic material adhered to a seat which can be firmed fitting to the first open end, having a piercing element and a central air passage, and packed separately in a package.
